# EUROPÄISCHE PATENTSCHRIFT

(11) **EP 1 012 152 B1**
(45) Veröffentlichungstag und Bekanntmachung des Hinweises auf die Patenterteilung: **09.10.2002**
(21) Anmeldenummer: 98945245.3
(22) Anmeldetag: 14.08.1998
(51) Int. Cl.: C07F 9/6584

(54) **VERFAHREN ZUR HERSTELLUNG VON OXAZAPHOSPHORIN-2-AMINEN**
METHOD FOR PREPARING OXAZAPHOSPHORIN-2-AMINES
PROCEDE POUR LA PREPARATION D'OXAZAPHOSPHORIN-2-AMINES

(30) Priorität: 06.09.1997 DE 19739159
(43) Veröffentlichungstag der Anmeldung: 28.06.2000
(73) Patentinhaber: Baxter Healthcare S.A., 83046 Wallisellen (CH)
(72) Erfinder: NIEMEYER, Ulf, D-33605 Bielefeld (DE); NIEGEL, Harald, D-01640 Coswig (DE); KUTSCHER, Bernhard, D-63477 Maintal 1 (DE); NEDA, Ion, D-38102 Braunschweig (DE)
(74) Vertreter: Hock, Joachim
(86) Internationale Anmeldenummer: EP9805170
(87) Internationale Veröffentlichungsnummer: WO99012942

(56) Entgegenhaltungen:
- CHEMICAL ABSTRACTS, vol. 093, no. 3, 21. Juli 1980 Columbus, Ohio, US; abstract no. 026467, SATO T: "Oxazaphospholine derivatives" XP002088312 & JP 54 157581 - (OTSUKA PHARMACEUTICAL CO., LTD.;JAPAN) 12. Dezember 1979
- CHEMICAL ABSTRACTS, vol. 107, no. 1, 6. Juli 1987 Columbus, Ohio, US; abstract no. 007385, "Preparation of ifosfamide" XP002088313 & ES 526 194 A (SOCIEDAD ESPANOLA ESPECIALIDADES FARMACO-TERAPEUTICAS S. A.;SPAIN) 16. April 1985
- CHEMICAL ABSTRACTS, vol. 101, no. 11, 10. September 1984 Columbus, Ohio, US; abstract no. 091231, "1,3,2-Oxaazaphosphorinane-2-oxide derivatives" XP002088314 & JP 59 031787 A (OTSUKA PHARMACEUTICAL CO., LTD.;JAPAN) 20. Februar 1984
- CHEMICAL ABSTRACTS, vol. 100, no. 5, 30. Januar 1984 Columbus, Ohio, US; abstract no. 034702, STEC W J ET AL: "Optically active derivatives of 1,3,2-oxazaphosphorinane" XP002088315 & PL 119 971 B (POLSKA AKADEMIA NAUK, CENTRUM BADAN MOLEKULARNYCH I MAKROMOLEKULARNYCH) 31. August 1983
- CHEMICAL ABSTRACTS, vol. 114, no. 11, 18. März 1991 Columbus, Ohio, US; abstract no. 102388, STEC W J ET AL: "Preparation of 2H-N-acyltetrahydro-2-oxo-1,3,2-oxazaphosp horines" XP002088316 & PL 149 593 B (INSTYTUT PRZEMYSLU FARMACEUTYCZNEGO;POL.) 28. Februar 1990

## Beschreibung

Die Erfindung betrifft ein Verfahren zur Herstellung von Oxazaphosphorin-2-aminen (N-substituierten Tetrahydro-2*H*-σ⁴λ⁵-1,3,2-oxazaphosphorin-2-amino-2-oxiden) der allgemeinen Formel 1, 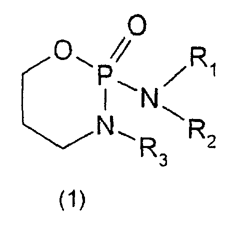 worin R₁ für H, 2-Bromethyl, 2-Chlorethyl, 2-Hydroxyethyl, 2-Mesyloxyethyl R₂ für H und 2-Chlorethyl und R₃ für H, 2-Bromethyl, 2-Chlorethyl stehen kann, oder R₁ und R₂ gemeinsam mit dem verknüpften N-Atom einen Aziridinring bilden. Die nach dem neuen Verfahren hergestellten Verbindungen sind entweder selbst Zytostatika oder Immunsuppressiva oder Ausgangsverbindungen für die Herstellung von racemischen und enantiomerenreinen Oxazaphosphorin-2-aminen mit zytostatischer oder immunsuppressiver Wirksamkeit.

Zu den Verbindungen der Formel 1 gehören die bekannten Arzneimittel Cyclophosphamid (R₁ = R₂ = 2-Chlorethyl, R₃ = H), lfosfamid (R₁ = R₃ = 2-Chlorethyl, R₂ = H) und Trofosfamid (R₁ = R₂ = R₃ = 2-Chlorethyl). Sie werden seit 1958 bzw. den 70er Jahren in der Krebstherapie verwendet (N. Brock. Cancer Res. 49, 1-7 (1989)). Ihre Synthese ist in den Patentschriften DE 1 057 119, GB 1 235 022 und DE 1 645 921 beschrieben. Weiterhin gehört zu den Verbindungen nach Formel 1 Sufosfamid (R₁ = 2-Mesyloxyethyl, R₂ = H, R₃ = 2-Chlorethyl), das als Immunsuppressivum für Autoimmunerkrankungen entwickelt wurde (DE 2 107 936, DOS 2 201 675), sowie Verbindungen, die sich zur Herstellung der genannten Oxazaphosphorin-2-amine in ihrer racemischen oder enantiomeren Form eignen (K. Pankiewicz et al., J. Amer. Chem. Soc. 101, 7712-7718 (1979) und K. Misiura et al. J. Med. Chem. 26, 674-679 (1983)).

Den literaturbekannten Synthesen ist gemeinsam, daß als phosphorhaltige Ausgangsverbindung 2-Chlor-tetrahydro-2H-1,3,2-oxazaphosphorin-2-oxid. Bis(2-chlorethyl)-phosphorsäureamid-dichlorid oder 2-Chlor-3-(2-chlorethyl)-tetrahydro-2H-1,3,2-oxazaphosphorin-2-amino-2-oxid in einem Syntheseschritt zum Oxazaphosphorin-2-amin umgesetzt wird. Die drei phosphorhaltigen Ausgangsverbindungen werden ihrerseits aus Phosphorylchlorid hergestellt (R.H. Iwamato et al. J. Org. Chem. 26, 4743-4745 (1961), O.M. Friedman et al., J. Amer. Chem. Soc. 76, 655-658 (1954) bzw. J.M.S. van Maanen et al.. J. Labelled Compd. Radiopharm. 18, 385-390 (1981)).

Diese Synthesen haben Nachteile. Die auf Phosphorylchforid bezogene Gesamtausbeute für die Oxazaphosphorin-2-amine bleibt wesentlich unter 50 % und ist daher für eine Zweistufensynthese relativ niedrig. Weiterhin ist die Isolierung der Zwischenstufe, d.h. eine der drei oben genannten phosphorhaltigen Ausgangsverbindungen, ungünstig, da sie thermisch und hydrolytisch instabil sind und während der Herstellung, Lagerung oder kurz vor dem Einsatz Anlaß zu Nebenreaktionen geben können. Außerdem werden die Vorteile einer zweistufigen *Eintopfreaktion* nicht genutzt.

Es bestand daher die Aufgabe. ein Verfahren zu realisieren, welches gegenüber dem bekannten Stand der Technik folgende Vorteile aufweist:
1. höhere Gesamtausbeute.
2. Vermeidung der Isolierung von Zwischenprodukten,
3. geringerer apparativer Aufwand,
4. verminderter Zeitbedarf,
5. geringerer Substanzbedarf.
6. Vermeidung chromatographischer oder anderer zusätzlicher Reinigungschritte und
7. reduzierter Zytostatika- bzw. Chemikalienabfall und damit geringere Umweltbelastung.

Die erfindungsgemäße Lösung des Problems besteht nun darin, daß ein Phosphorylhalogenid und zwei Amine unter Minimierung des Einflusses von Wasser und von Alkoholen sowie ohne Isolierung einer Zwischenverbindung zu den Verbindungen der allgemeinen Formel 1 in einem inerten organischen Lösungsmittel und mit einer Hilfsbase umgesetzt werden. in der Regel wird das Verfahren als zweistufige Eintopfreaktion, bei dem die Reaktionsteilnehmer nacheinander ins Reaktionsgefäß gegeben werden, durchgeführt.

Für die erfolgreiche Durchführung des neuen Verfahrens ist entscheidend, daß der Einfluß von Wasser, insbesondere auf Phosphorylchlorid, zurückgedrängt wird. Aufgrund der ausgiebigen Untersuchung der Erfinder wurde nachgewiesen, daß mit steigendem Wassergehalt im Reaktionsgemisch die Anzahl und Menge von Nebenprodukten zunimmt und die Ausbeute an den Zielverbindungen drastisch vermindert wird. Die Kristallisation der Endprodukte ist dann nicht mehr oder nur nach zusätzlichen Reinigungsschritten, z.B. Chromatographie, möglich. Dieser überraschende Einfluß von Wasser auf den Syntheseerfolg wurde bislang noch nicht in der Literatur berücksichtigt. Statt dessen wurde den Schwierigkeiten einer zweistufigen Eintopfsynthese, die von Phosphorylchlorid ausgeht, aus dem Wege gegangen. Man wählte die nur vordergründig vorteilhafte einstufige Synthese, bei der man eine der drei oben genannten phosphorhaltigen Ausgangsverbindungen verwendet. Im Chemical Abstracts Vol.100, No. 5, 34702 Columbus Ohio, US ist ferner die Herstellung von optisch aktiven Oxazaphosphorinen beschrieben (PL-A-119 971). Die Synthese erfaßt drei bis fünf Syntheseschritte und führt schließlich zu dem optisch aktiven und cytostatisch aktiven Wirkstoffen. Die Zwischenstufen werden mit Ausnahme der ersten Stufe isoliert. Die in der ersten und zweiten Stufe hergestellten Verbindungen sind optisch aktive Phenylethylamin- bzw. Naphthylethylamin-oxaphosphorine, die nicht Gegenstand der vorliegenden Erfindung sind.

Neben Wasser ist auch auf Abwesenheit von Alkoholen zu achten. So ließ sich zeigen, daß Spuren von Methanol und Ethylalkohol im Lösungsmittel zu Nebenreaktionen und Ausbeuteminderung führen können.

Die Phosphorylhalogenide haben die allgemeine Formel 2 und die zwei Amine die allgemeine Formel 3 und 4: 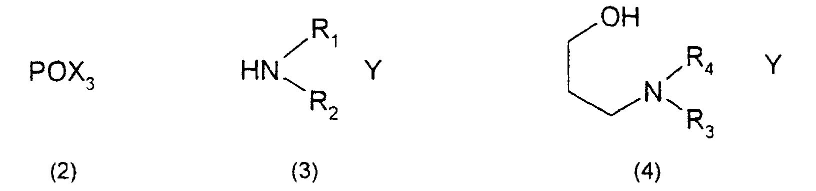 worin R₁, R₂ und R₃ die gleiche Bedeutung wie in Formel 1 haben, R₄ für H stehen kann, oder R₃ und R₄ gemeinsam mit dem verknüpften N-Atom einen Aziridinring bilden, X für Chlor und Brom steht, wobei X nur Brom ist, wenn R₁ oder R₃ für 2-Bromethyl steht, und Y keine Bedeutung hat oder für Chlorwasserstoff und Bromwasserstoff stehen kann.

Die Umsetzung wird in inerten organischen Lösungsmitteln oder Lösungsmittelgemischen durchgeführt, wobei beispielsweise Dichlormethan, 1,2-Dichlorethan, Chloroform. Dioxan. Acetonitril, Tetrahydrofuran und Toluol in Frage kommen.

Als Hilfsbase, bzw. als säurebindendes Mittel ist beispielsweise Triethylamin, Pyridin, und Natriumcarbonat geeignet.

Die Konzentration der Reaktionsmischung, d.h. das Verhältnis von Verbindung der Formel 2, 3 bzw. 4 zum Volumen des Lösungsmittels, kann zwischen 0,1 bis 6 Mol pro Liter variieren.

Die Amine der allgemeinen Formel 3 und 4 werden in der Regel equimolar bezogen auf die Verbindung der Formel 2 eingesetzt, wobei ein bis zu 5 prozentiger Unterschuß oder 20 prozentiger Überschuß möglich ist.

Die Hilfsbase wird in der Regel in equivalenter Menge zum Amin eingesetzt, d.h. für die Kondensation der Verbindung der Formel 3 werden zwei Equivalente Hilfsbase benötigt, wenn sie als Salz vorliegt, und ein Equivalent Hilfsbase, wenn sie als freie Base eingesetzt wird. Für die Verbindungen der Formel 4 werden entsprechend drei bzw. zwei Equivalente gebraucht. Ein bis zu 30 prozentiger Überschuß an Hilfsbase ist möglich.

Die Kondensationsreaktion des Phosphoryihalogenids mit den Aminen verläuft exotherm. Zur Steuerung der Reaktionstemperatur wird die Reaktionskomponente, mit der die Kondensation ausgelöst wird, langsam unter Kühlung zugegeben. Deshalb wird beispielsweise Phosphorylchlorid langsam zu einem Amin. das als freie Base vorliegt. zugetropft oder die Hilfsbase wird langsam zu einer Mischung aus Phosphorylchlorid und einem Amin, das als Salz vorliegt, gegeben. Die Reaktionstemperatur wird in der Anfangsphase in der Regel durch Kühlung im Temperaturbereich von -40 °C bis 20 °C. insbesondere zwischen - 20 und + 10 °C, gehalten. Sie kann nach der Hälfte der Umsetzung bis 100 °C bzw. bis zur Siedetemperatur des Lösungsmittels - ggf. durch Heizen - steigen.

Die Verbindungen der Formel 2 bis 4 und die Hilfsbase werden zu bestimmten Zeitpunkten zusammengegeben. Der folgende Standardansatz mit seinen Variationen soll dies verdeutlichen.

Ein Amin der Formel 3 und die equivalente Menge Hilfsbase werden im Lösungsmittel vorgelegt, und eine Verbindung der Formel 2 läßt man langsam zutropfen bzw. zulaufen (erster Schritt). Anschließend wird die Verbindung der Formel 4 zugegeben und dann die Hilfsbase langsam dosiert (zweiter Schritt).

Dieser Standardansatz kann im ersten Schritt so variiert werden, daß Verbindung der Formel 2 vorgelegt wird und dann das Amin der Formel 3 gemeinsam mit der Hilfsbase langsam zugegeben wird. Weiterhin kann das Amin der Formel 3 in seiner Salzform im Lösungsmittel vorgelegt, Verbindung der Formel 2 zugegeben und dann die Hilfsbase langsam zudosiert werden. Desweiteren können Hilfsbase und Verbindung der Formel 2 getrennt gleichzeitig langsam zum vorgelegten Amin der Formel 3 zugegeben werden, wobei beide Zugaben so gegeneinander verschoben sein sollten, daß die Hilfsbase überschüssig zur Verbindung der Formel 2 vorliegt.

Im zweiten Schritt kann die Verbindung der Formel 4 auch gemeinsam mit der Hilfsbase langsam zugegeben werden; oder die Verbindung der Formel 4 und die Hilfsbase wird vorgelegt und das Reaktionsgemisch aus dem ersten Schritt wird langsam zugegeben.

Der Standardansatz kann so variiert werden, daß das Amin der Formel 4 im ersten Schritt und das Amin der Formel 3 im zweiten Schritt eingesetzt wird. Die gesamte Hilfsbase für beide Stufen kann auch im ersten Schritt eingesetzt werden. Auch die gleichzeitige, langsame Zugabe der beiden Amine mit der Hilfsbase zu dem vorgelegten Phosphorylchlorid ist möglich.

Auch andere als hier beschriebene Zeitpunkte für die Zugabe der vier Komponenten sind für das Verfahren möglich. Es ist aber beispielsweise ungünstig, Phosphorylchlorid zusammen mit Triethylamin vorzulegen oder 2-Chlorethylamin Hydrochlorid zusammen mit der Hilfsbase vorzulegen und Phosphorylchlorid zuzugeben.

Wird die Umsetzung in zwei Schritten durchgeführt, so wird nach dem ersten Schritt in der Regel 30 Minuten oder länger (häufig zweckmäßig über Nacht) nachgerührt. Dadurch reagiert Phosphorylchlorid zu definierten, literaturbekannten Zwischenverbindungen. So entsteht mit 3-Amino-1-propanol die Verbindung 2-Chlor-tetrahydro-2H-1,3,2-oxazaphosphorin-2-amino-2-oxid, mit Bis(2-chlorethyl)amin Hydrochlorid die Verbindung Bis-N,N-(2-chlorethyl)-phosphorsäureamid-dichlorid, mit N-(2-Chlorethyl)-3-hydroxypropylamin Hydrochlorid bzw. 3-N-Hydroxypropylaziridin die Verbindung 2-Chlor-3-(2-chlorethyl)-tetrahydro-2H-1,3,2-oxazaphosphorin-2-amino-2-oxid und mit 2-Chlorethylamin Hydrochlorid die Verbindung N-(2-chlorethyl)-phosphorsäureamid-dichlorid.

Zur Verringerung von Wasser im Reaktionsmedium werden alle Einsatzstoffe und -geräte möglichst wasserfrei verwendet. So werden beispielsweise Geräte zur Entfemung von Restfeuchtigkeit ausgeheizt, vorgetrocknete Lösungsmittel eingesetzt, die hygroskopischen Amine der Formel 3 und 4 kurz vor dem Einsatz getrocknet, die Phosphorylhalogenide unter trockenen Bedingungen frisch destilliert und die Hilfsbase entsprechend vorgetrocknet. Die Reaktionslösung sollte vor dem Start der Umsetzung einem Wassergehalt unter 0,5 %. besser jedoch unter 0,1 % bzw. unter 0,001 % besitzen.

Die Bildung von Nebenprodukten läßt sich auch vermindern, wenn die Reaktionsgemisch vor dem Start der Umsetzung mit Zusätzen behandelt wird. Solche Zusätze können beispielsweise Molekularsieb, Aluminiumoxid in seinen verschiedenen Formen, Calciumchlorid (wasserfrei oder eine Hydratform). Phosphorpentoxid und Magnesiumchlorid sein. In der Regel liegt der Zusatz zwischen 5 g und 150 g pro Mol eingesetzte Verbindung der Formel 2 und die Lösung bzw. Suspensionen wird eine halbe bis 3 Stunden gerührt, bevor die Kondensationsreaktion durch Zugabe der Hilfsbase bzw. des Phosphorylhalogenids gestartet wird. Dabei ist es günstig, in homogener Phase zu arbeiten. Beispielsweise gehen 3,9 g N-2-Chlorethyl-3-hydroxypropylamin Hydrochlorid in 60 ml Dichlormethan nach Zugabe von 3,2 ml Triethylamin vollständig in Lösung (s. Beispiel 3).

Nachdem das erste oder auch zweite Chloratom der Phosphorylhalogenids reagiert hat, besteht die Möglichkeit die ausgefallenen Salze abzutrennen oder das Reaktionsgemisch zu waschen und danach die Umsetzung durch Zugabe von Amin und Hilfsbase zu vervollständigen.

Die Aufarbeitung des Reaktionsgemisches am Ende der Reaktion erfolgt durch Abfiltrieren der Salze, und / oder durch Waschen mit Wasser bei verschiedenen pH-Werten. Dabei ist die Hydrolysierbarkeit und Wasserlöslichkeit der Oxazaphosphorin-2-amine zu berücksichtigen, z.B. durch kurze Kontaktzeit mit der Säure oder Kühlung des Ansatzes. Auch die wasserfreie Aufarbeitung des Reaktionsgemisches ist günstig oder die Neutralisation der Reaktionslösung durch HCI-Gas auf pH 4 bis 6. Desweiteren kommt die Verwendung von schwachen Säuren, z.B. Essigsäure und Oxalsäure, in Frage.

Die Amine der Formel 3 und 4 können prinzipiell auch in nicht reiner Form eingesetzt werden. Beispielsweise läßt sich N-(2-Chlorethyl)-3-hydroxypropylamin Hydrochlorid auch in Gegenwart von 15% N-(2-Chlorethyl)-3-chlorpropylamin Hydrochlorid zur Synthese einer Verbindung der Formel 1 verwenden (s. Beispiel 2). Zu erwarten war eine mehr als 10 %ige Nebenreaktion, so daß die Gewinnung der Verbindung der Formel 1 erschwert oder gar verhindert wird. Überraschend trat nur ein minimaler Ausbeuteverlust ein. Die Reinigung von N-(2-Chlorethyl)-3-hydroxypropylamin Hydrochlorid, die sehr aufwendig ist, kann daher unterbleiben. Dies ist ein wesentlicher Synthesevorteil.

Zu den erfindungsgemäßen Verbindungen gehören auch Oxazaphosphorin-2-amine, die sich für die Umsetzung zu Cyclophosphamid. Ifosfamid, Trofosfamid und Sufosfamid eignen. So ist die Vorstufe von Sufosfamid, d.h. N-(2-Chlorethyl)-2-(2-hydroxyethyl)tetrahydro-2H-1.3,2-oxazaphosphorin-2-amino-2-oxid, zugänglich und läßt sich direkt weiter zu Sufosfamid mesylieren (s. Beispiel 7).

Die Ausbeute für die erfindungsgemäßen Verbindungen der Formel 1 konnte beträchtlich verbessert werden. Dies wird im folgenden anhand des Vergleichs zwischen der auf Phosphorylchlorid bezogenen Gesamtausbeute, die sich aus bisher publizierten Ausbeuten (Literatur siehe oben) zu den beiden Stufen berechnen läßt, und der Ausbeute für die Beispiele 3, 5, 6 bzw. 8 zeigen.

So ist die auf Phosphorylchlorid bezogene Ausbeute für 2-Chlor-3-(2-chlorethyl)-tetrahydro-2H-1,3,2-oxazaphosphorin-2-amino-2-oxid 49 % (J.M.S. van Maanen. Seite 388). Die auf 2-Chlor-3-(2-chlorethyl)-tetrahydro-2H-1,3,2-oxazaphosphorin-2-amino-2-oxid bezogene Ausbeute für Ifosfamid ist 71 % (DE 1 645 921: Beispiel 4, Spalte 6 und 7), so daß sich eine Gesamtausbeute von 35 % Ifosfamid berechnen läßt. Folglich ist die Ausbeute nach dem neuen Verfahren (Beispiel 3, Ausbeute 73 %) doppelt so hoch.

Die auf Phosphorylchlorid bezogenene Ausbeute für Bis(2-chlorethyl)-phosphorsäureamiddichlorid beträgt 16 % (O.M. Friedman, S. 657). Die auf Bis(2-chlorethyl)-phosphorsäureamiddichlorid bezogene Ausbeute für Cyclophosphamid Monohydrat ist 65-70 % (DE 1 057 119: Beispiel 10, Spalte 9), so daß sich eine Gesamtausbeute von 11 % berechnen läßt. Die Ausbeute nach dem neuen Verfahren (Beispiel 6, 72 % Ausbeute an Cyclophosphamid Monohydrat) ist also beträchtlich höher.

Die auf 2-Chlor-3-(2-chlorethyl)-tetrahydro-2H-1,3,2-oxazaphosphorin-2-amino-2-oxid bezogene Ausbeute für die Verbindung der allgemeinen Formel 1, worin R₁ für 1-Phenylethyl, R₂ für H und R₃ für 2-Chlorethyl steht, ist 68 % (ein 1:1-Diasteromerengemisch. K. Misiura, S. 677), so daß sich eine Gesamtausbeute von 34 % ergibt. Die Ausbeute nach dem neuen Verfahren ist mit 66 % also fast doppelt so hoch (Beispiel 5), so daß auch (R)-lfosfamid unter Nutzung des neuen Verfahrens mit fast doppelter Ausbeute zugänglich ist.

Sufosfamid wurde bisher ausgehend von 2-Chlor-3-(2-chlorethyl)-tetrahydro-2H-1,3,2-oxazaphosphorin-2-amino-2-oxid mit einer Ausbeute von 30 % hergestellt (DE 2 107 936: Beispiel 9, Spalte 8 und 9), so daß sich eine auf Phosphorylchlorid bezogene Gesamtausbeute von 15 % berechnen läßt. Nach dem neuen Verfahren beträgt die Ausbeute an Sufosfamid 38 % (Beispiel 8) und ist also mehr als doppelt so hoch.

Neben der Ausbeuteverbesserungen sind auch die anderen oben erwähnten, angestrebten Vorteile erreicht worden. So ist für Sufosfamid keine chromatographische Reinigung mehr erforderlich. Ifosfamid läßt sich gemäß Beispiel 1 so herstellen, daß im 1. Schritt 2-Chlorethylamin Hydrochlorid und im 2. Schritt N-(2-Chlorethyl)-3-hydroxypropylamin Hydrochlorid eingesetzt wird. Dieses Verfahren war bisher nicht möglich. Die Isolierung der Zwischenstufe wird in allen Beispielen vermieden, wodurch entscheidende Vereinfachungen bzw. Verfahrensverbesserung erreicht werden. Das neue Verfahren ist für die technische Synthese in ökonomischer als auch ökologischer Hinsicht ein enorm vorteilhaftes Verfahren.

Die folgenden Beispiele dienen der weiteren Erläuterung der vorliegenden Erfindung, ohne sie hierauf zu beschränken.

### Beispiel 1

### N,3-Bis(2-chlorethyl)tetrahydro-2H-1,3,2-oxazaphosphorin-2-amino-2-oxid (Ifosfamid)

In einer auf ca. 0 °C abgekühlten Lösung von 5,52 g Phosphorylchlorid in 80 ml Dichlormethan werden 4,18 g 2-Chlorethylamin Hydrochlorid suspendiert und unter Rühren bei 0 - 10 °C innerhalb von 1 Stunde 10 ml Triethylamin getropft. Anschließend werden zum Reaktionsgemisch 6,3 g N-(2-Chlorethyl)-3-hydroxypropylamin Hydrochlorid gegeben und unter Rühren 15 ml Triethylamin zugetropft. Nach 6 Stunden Rühren bei Raumtemperatur wird mit 2mal mit 10 ml verdünnter Salzsäure (Waschwasser mit >pH 2), mit 10 ml verdünnter Sodalösung und 2mal mit 10 ml Wasser ausgeschüttelt. Die organische Phase wird über Natriumsulfat getrocknet und im Vakuum eingeengt. Anschließend wird in 100 ml Diethylether aufgenommen, über Kohle filtriert und eingeengt. Der Rückstand wird in 30 ml Diethylether gelöst und zur Kristallisation bei -5 °C aufbewahrt. Am nächsten Tag wird abgesaugt und getrocknet.
Ausbeute 5,3 g (56 % der Theorie), Schmelzpunkt: 48 - 51 °C
Dünnschichtchromatographie mit DC-Fertigplatten. Kieselgel 60_{F245} der Fa. Merck. Diese DC-Methode wurde auch bei den folgenden Beispielen verwendet. R_{F}-Wert = 0.58 (CH₂Cl₂ : CH₃OH = 90 : 10), R_{F}-Wert = 0,30 (CH₂Cl₂: CH₃OH = 95 : 5)
¹H-NMR (500 MHz, CDCl₃): δ = 1,9-2,0 (m, 2H, CH₂), 3,2-3,5 (m, 7H, 3 CH₂, NH), 3.6 (t, 2H, CH₂-Cl), 3,6-3,7 (m, 2H, CH₂-Cl), 4,2-4,4 (m, 2H, CH₂-O)
³¹P-NMR (202 MHz, CDCl₃): δ = 12.6

### Beispiel 2

### N,3-Bis(2-chlorethyl)tetrahydro-2H-1,3,2-oxazaphosphorin-2-amino-2-oxid (Ifosfamid)

Zu 300 ml Dichlormethan werden unter Rühren bei 0 °C - 10 °C 20,4 g N-(2-Chlorethyl)-3-hydroxypropylamin Hydrochlorid (85 %ig) und 42 ml Triethylamin gegeben. Anschließend werden 15,3 g Phosphorylchlorid unter Rühren zugetropft. Nach einer Stunde bei Raumtemperatur wird unter Kühlen bei 15 °C 12,8 g 2-Chlorethylamin Hydrochlorid und 30,8 ml Triethylamin zugegeben und über Nacht bei Raumtemperatur gerührt. Die Aufarbeitung erfolgt wie beim Beispiel 1. Die Kristallisation wird in tert.-Butyl-methylether durchgeführt.
Ausbeute: 17 g (65 % der Theorie). Schmelzpunkt: 47 - 50°C
R_{F}-Werte und NMR-Daten entsprechen denen vom Beispiel 1.

### Beispiel 3

### N,3-Bis(2-chlorethyl)tetrahydro-2H-1.3.2-oxazaphosphorin-2-amino-2-oxid (Ifosfamid)

3,87 g N-(2-Chlorethyl)-3-hydroxypropylamin Hydrochlorid (90 %ig), 0,2 g Calciumchlorid und 3,2 ml Triethylamin werden in 60 ml Dichlormethan, das ca. 0,1 % Wasser enthält, 60 min bei 0 °C gerührt. Anschließend werden gleichzeitig 6.0 ml Triethylamin mit einer Tropfgeschwindigkeit von 1 ml/min und 3,07 g Phosphorylchlorid mit einer Tropfgeschwindigkeit von 0,3 ml/min bei maximal 5 °C zugetropft. Anschließend wird noch 2 Stunden bei 0 °C gerührt und mit 2,5 g 2-Chlorethylamin Hydrochlorid versetzt. Nach Zugabe von 5,6 ml Triethylamin wird noch 10 Stunden bei Raumtemperatur gerührt, die Reaktionslösung durch Einleiten von HCI-Gas auf pH 4-6 gebracht, einmal mit 12 ml Wasser und zweimal mit 2,5 ml Sodalösung ausgeschüttelt. Weitere Aufarbeitung wie beim Beispiel 1.
Ausbeute: 3,9 g (73 % der Theorie), Schmelzpunkt: 49 - 51 °C
R_{F}-Werte und NMR-Daten entsprechen denen vom Beispiel 1.

### Beispiel 4

### N3-Bis(2-chlorethyl)tetrahydro-2H-1,3,2-oxazaphosphorin-2-amino-2-oxid (Ifosfamid)

Zu 200 ml Dichlormethan werden unter Rühren bei 0 °C 15,3 g Phosphorylchlorid gegeben und unter Rühren mit einer Lösung von 10 g N-3-Hydroxypropylaziridin in 14,6 ml Triethylamin bei 0 bis 20 °C versetzt. Am nächsten Tag werden 12 g 2-Chlorethylamin Hydrochlorid zugesetzt und anschließend 29 ml Triethylamin zugetropft. Die Aufarbeitung erfolgt wie beim Beispiel 1.
Ausbeute: 18 g (69 % der Theorie). Schmelzpunkt: 47 - 50°C
R_{F}-Werte und NMR-Daten entsprechen denen vom Beispiel 1.

### Beispiel 5

### N,N-Bis(2-chlorethyl)tetrahydro-2H-1,3,2-oxazaphosphorin-2-amino-2-oxid, Monohydrat (Cyclophosphamid Monohydrat)

Zu einer Suspension von 17,8 g N,N-Bis(2-chlorethyl)amin Hydrochlorid in 200 ml Dichlormethan 15,3 g Phosphorylchlorid gegeben und anschließend bei 0 bis 10 °C 29 ml Triethylamin zugetropft. Nach 3 Stunden wird eine Mischung aus 7,6 ml 3-Amino-1-propanol und 28 ml Triethylamin bei maximal 15 °C zugegeben. Am nächsten Tag wird mit 80 ml Eiswasser gewaschen, die wäßrige Phase mit Dichlormethan extrahiert, die vereinigten Dichlormethan-Phasen eingeengt, mit Diethylether aufgenommen, mit Aktivkohle behandelt, filtriert und mit Wasser gesättigt. Nach der Kristallisation bei 0 °C wurde abgesaugt und getrocknet.
Ausbeute: 20 g (72 % der Theorie). Schmelzpunkt 50-52 °C
R_{F}-Wert = 0,58 (CH₂Cl₂ : CH₃OH = 90 : 10), R_{F}-Wert = 0,24 (CH₂Cl₂ : CH₃OH = 95 : 5) ¹H-NMR (500 MHz, CDCl₃): δ = 1.8-2,0 (m, 2H, CH₂), 2,6 (H₂O), 3,2-3,6 (m, 7H, 3 CH₂-N, NH), 3,6-3,7 (t, 4H, CH₂-Cl), 4,2-4,3 (m, 1H, CH₂-O), 4,3-4,5 (m, 1H, CH₂-O)
³¹P-NMR (202 MHz, CDCl₃): δ = 13.0

### Beispiel 6

### N,N-Bis(2-chlorethyl)tetrahydro-2H-1,3,2-oxazaphosphorin-2-amino-2-oxid, Monohydrat (Cyclophosphamid Monohydrat)

Zu einer Lösung von 7,6 g 3-Amino-1-propanol und 28 ml Triethylamin in 200 ml Dichlormethan werden 15,3 g Phosphorylchlorid unter Rühren bei 2 bis 15 °C gegeben. Nach 1 Tag bei Raumtemperatur wird 17.8 g N,N-Bis(2-chlorethyl)amin Hydrochlorid bei 10 bis 20 °C 29 ml Triethylamin zugetropft. Anschließend wird die Lösung mehre Stunden unter Rückfluß erwärmt. Die Aufarbeitung wird wie beim Beispiel 4 durchgeführt und der Rückstand aus Ethanol/Wasser (3:5) umkristallisiert.
Ausbeute: 19 g (68 % der Theorie), Schmelzpunkt 49-52 °C
R_{F}-Werte und NMR-Daten entsprechen denen vom Beispiel 5.

### Beispiel 7

### N-(2-Chlorethyl)-2-(2-mesyloxyethyl)tetrahydro-2H-1,3,2-oxazaphosphorin-2-amino-2-oxid (Sufosfamid)

26,1 g N-(2-Chlorethyl)-3-hydroxypropylamin Hydrochlorid und 23,0 g Phosphorylchlorid werden in 200 ml Dichlormethan suspendiert und unter starkem Rühren bei 0 bis 5 °C mit 64 ml Triethylamin versetzt. Anschließend wird 2 Stunden bei Raumtemperatur gerührt, der Niederschlag abfiltriert und mit Dichlormethan gewaschen, die vereinigten organische Phasen mit Eiswasser gewaschen und über Natriumsulfat getrocknet, mit Aktivkohle behandelt und filtriert. Eine Lösung von 8,8 g Ethanolamin in 21 ml Triethylamin wird unter Rühren bei 15 bis 20 °C dazugegeben und 3 Stunden bei Raumtemperatur nachgerührt. Anschließend werden 20 ml Triethylamin zugegeben und bei Raumtemperatur 16,6 g Methansulfonsäurechlorid zugetropft. Die Salze werden abfiltriert und mit Dichlormethan gewaschen. Die vereinigten organischen Phasen werden 4 mal mit gesättigter Kochsalz-Lösung gewaschen und die vereinigten wäßrigen Phasen mit Dichlormethan extrahiert. Anschließend werden die vereinigten organischen Phasen mit Natriumsulfat getrocknet, mit Aktivkohle behandelt und eingeengt. Der Rückstand wird in wenig Dichlormethan aufgenommen, mit Diethylether versetzt und zur Kristallisation in den Kühlschrank gestellt.
Ausbeute: 18 g (38 % der Theorie), Schmelzpunkt: 77 - 79 °C
R_{F}-Wert = 0,60 (CH₂Cl₂: CH₃OH = 90 : 10), R_{F}-Wert = 0,18 (CH₂Cl₂: CH₃OH = 95 : 5)
¹H-NMR (500 MHz, CDCl₃): δ = 1.9 (m, 2H, CH₂), 3,1 (s, 3H, CH₃), 3,2-3,4 (m, 5H, CH₂-N), 3,4-3,5 (m, 1H, CH₂-N), 3,7 (t, 2H, CH₂-Cl), 3.7-3.8 (m, 1H. NH), 4,2-4,3 (m, 3H, CH₂-OP, CH₂-OS), 4,3-4,2 (m, 1H, CH₂-OP
³¹P-NMR (202 MHz, CDCl₃): δ = 12,4

### N-(2-Chlorethyl)-2-(2-hydroxyethyl)tetrahydro-2H-1,3.2-oxazaphosphorin-2-amino-2-oxid (Vorstufe für die Mesylierung von Sufosfamid)

R_{F}-Wert = 0,45 (CH₂Cl₂ : CH₃OH = 90 : 10)
¹H-NMR (500 MHz, CDCl₃): δ = 1,9-2,0 (m, 2H. CH₂), 3,0-3,1 (m, 2H, CH₂-N), 3,2-3,3 (m, 3H, CH₂-N), 3,4-3,5 (m, 1H, CH₂-N), 3,6-3,7 (t, 2H, CH₂-N), 3,6-3,7 (m, 4H, CH₂-Cl, CH₂O), 4,0-4,1 (m, 1H, NH), 4,2-4,3 (m, 1H, CH₂-OP), 4,3-4,4 (m, 1H, CH₂-O), 4,1-4.8 (1H, OH)
¹H-NMR (500 MHz, DMSO-D₆): δ = 1,7-1,8 (m, 1H, CH₂), 1,8-1,9 (m, 1H, CH₂), 2,7-2,8 (m, 2H, CH₂-N), 3,1-3,3 (m, 4H, CH₂-N), 3,4-3,5 (m, 1H, CH₂-O), 3,6-3,7 (t, 2H, CH₂-Cl), 4,1-4,2 (m, 2H, CH₂-OP), 4,5-4,6 (m, 2H, CH₂-O), 4,5-4.8 (1H, OH)
³¹P-NMR (202 MHz, CDCl₃): δ = 14.2

### Beispiel 8

### N-(2-Chlorethyl)-2-(2-mesyloxyethyl)tetrahydro-2H-1,3,2-oxazaphosphorin-2-amino-2-oxid (Sufosfamid)

26,1 g N-(2-Chlorethyl)-3-hydroxypropylamin Hydrochlorid und 23,0 g Phosphorylchlorid werden in 200 ml Dichlormethan suspendiert und unter starkem Rühren bei 0 bis 5 °C mit 64 ml Triethylamin versetzt. Anschließend wird 2 Stunden bei Raumtemperatur gerührt. Eine Lösung von 8,8 g Ethanolamin in 21 ml Triethylamin wird unter Rühren bei 15 bis 20 °C dazugegeben und 3 Stunden bei Raumtemperatur nachgerührt. Die Mesylierung und Aufarbeitung erfolgt analog zum Beispiel 8.
Ausbeute: 17 g (36 % der Theorie), Schmelzpunkt: 77 - 79 °C
R_{F}-Werte und NMR-Daten entsprechen denen vom Beispiel 7.

### Beispiel 9

### N,N-Bis(2-chlorethyl)-3-(2-chlorethyl)tetrahydro-2H-1,3,2-oxazaphosphorin-2-amino-2-oxid (Trofosfamid)

Zu einer Suspension von 17,8 g N,N-Bis(2-Chlorethyl)amin Hydrochlorid in 200 ml Dichlormethan werden 15,3 g Phosphorylchlorid gegeben und anschließend bei 0 bis 10 °C 28 ml Triethylamin zugetropft. Nach 3 Stunden werden unter Rühren 17,4 g N-(2-Chlorethyl)-3-hydroxypropylamin Hydrochlorid und 42 ml Triethylamin bei maximal 25 °C zugegeben. Die Reaktionsmischung wird 10 Stunden auf Rückfluß erhitzt. Am nächsten Tag wird analog zu Beispiel 1 gewaschen. Der ölige Rückstand wird in Diethylether aufgenommen, mit Aktivkohle behandelt, eingeengt und in wenig Diethylether aufgenommen und bei -10 bis 0 °C kristallisiert.
Ausbeute: 17 g (54 % der Theorie). Schmelzpunkt 50-52 °C
R_{F}-Wert = 0,85 (CH₂Cl₂ : CH₃OH = 90 : 10), R_{F}-Wert = 0,42 (CH₂Cl₂ : CH₃OH = 95 : 5)
¹H-NMR (500 MHz, CDCl₃): δ = 1,8-1,9 (m, 1H, CH₂), 2,0-2,1 (m, 1H, CH₂), 3,2-3,4 (m, 6H, 3 CH₂-N), 3,4-3,6 (m, 2H, CH₂-N), 3,6-3,8 (m, 6H. 3 CH₂-Cl), 4,2-4,3 (m, 1H, CH₂-O), 4,3-4,4 (m. 1H, CH₂-O)
³¹P-NMR (202 MHz, CDCl₃): δ = 13,8

### Beispiel 10

### N,N-Bis(2-chlorethyl)-3-(2-chlorethyl)tetrahydro-2H-1,3,2-oxazaphosphorin-2-amino-2-oxid (Trofosfamid)

Zu einer Suspension von 17,4 g N-(2-Chlorethyl)-3-hydroxypropylamin Hydrochlorid in 400 ml Dichlormethan werden 15,3 g Phosphorylchlorid gegeben und anschließend bei 5 bis 20 °C 46 ml Triethylamin zugetropft. Nach 3 Stunden werden unter Rühren 17,8 g N,N-Bis(2-Chlorethyl)amin Hydrochlorid und 29 ml Triethylamin zugegeben. Die Reaktionsmischung wird mehrere Stunden auf Rückfluß erhitzt. Anschließend wird auf Raumtemperatur abgekühlt, die Dichlormethanphase mehrfach mit Wasser gewaschen und die getrocknete organische Phase eingeengt. Der ölige Rückstand wird wie im Beispiel 10 zur Kristallisation gebracht.
Ausbeute: 21 g (64 % der Theorie). Schmelzpunkt 50-52 °C
R_{F}-Werte und NMR-Daten entsprechen denen vom Beispiel 9.

## Patentansprüche

1. Verfahren zur Herstellung von Oxazaphosphorin-2-aminen der allgemeinen Formel 1, 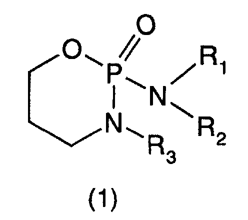 worin R₁ für H, 2-Bromethyl, 2-Chlorethyl, 2-Hydroxyethyl, 2-Mesyloxyethyl, R₂ für H und 2-Chlorethyl und R₃ für H, 2-Bromethyl, 2-Chlorethyl stehen kann, oder R₁ und R₂ gemeinsam mit dem verknüpften N-Atom einen Aziridinring bilden, wobei R₁, R₂ und R₃ nicht gleichzeitig H ist,
**dadurch gekennzeichnet, daß** man ein Phosphorylhalogenid der allgemeinen Formel 2,
ein Amin der allgemeinen Formel 3
und ein Amin der allgemeinen Formel 4, 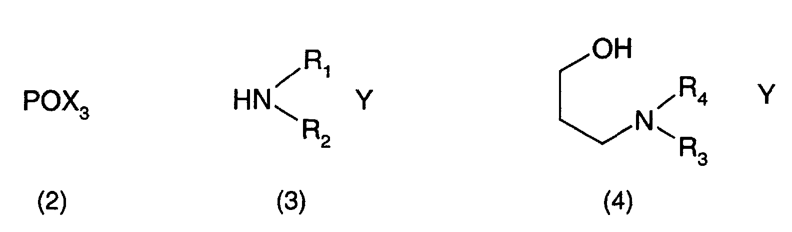 , worin R₁, R₂ und R₃ die gleiche Bedeutung wie in Formel 1 haben, R₄ für H stehen kann, oder R₃ und R₄ gemeinsam mit dem verknüpften N-Atom einen Aziridinring bilden, X für Chlor und Brom steht, wobei X nur Brom ist, wenn R₁ oder R₃ für 2-Bromethyl steht, und Y keine Bedeutung hat oder für Chlorwasserstoff und Bromwasserstoff stehen kann,
mit einer Hilfsbase als säurebindendes Mittel, wie Pyridin, Triethylamin, Alkali- und Erdalkalicarbonate, in Gegenwart von inerten Lösungs- oder Verdünnungsmitteln oder Gemischen, wobei z.B. halogenierte und nichthalogenierte Kohlenwasserstoffe, aromatische und aliphatische Kohlenwasserstoffe, wie insbesondere Toluol, Chloroform und Dichlormethan, aliphatische und zyklische Ether, wie insbesondere Diethylether und tert.-Butyl-methylether, Dioxan, Tetrahydrofuran, eingesetzt werden, unter Minimierung des Einflusses von Wasser und Alkoholen und ohne Isolierung einer Zwischenverbindung umsetzt.

2. Verfahren nach Anspruch 1, **dadurch gekennzeichnet, daß** im ersten Schritt je eine Verbindung der Formel 2 und 3 und im zweiten Schritt eine Verbindung der Formel 4 eingesetzt wird, wobei R₁ nicht gleich 2-Hydroxyethyl ist, wenn die Verbindung der Formel 3 im ersten Schritt eingesetzt wird.

3. Verfahren nach Anspruch 1 oder 2, **dadurch gekennzeichnet, daß** eine Verbindung der allgemeinen Formel 3 als Salz zusammen mit einer Verbindung der Formel 2 vorgelegt wird und die Hilfsbase langsam unter Kühlung zugegeben wird.

4. Verfahren nach Anspruch 2 oder 3, **dadurch gekennzeichnet, daß** die Verbindung der Formel 4 anstelle der Verbindung der Formel 3 und die Verbindung der Formel 3 anstelle der Verbindung der Formel 4 eingesetzt wird.

5. Verfahren nach Anspruch 1, **dadurch gekennzeichnet, daß** die Verbindungen der Formel 3 und 4 gleichzeitig oder gegeneinander verschoben mit Verbindung der Formel 2 kondensiert werden.

6. Verfahren nach einem der vorstehenden Ansprüche, **dadurch gekennzeichnet, daß** man die Verbindung N-(2-Chlorethyl)-3-hydroxypropylamin Hydrochlorid, die N-(2-Chlorethyl)-3-chlorpropylamin Hydrochlorid enthält, als Ausgangsverbindung einsetzt.

7. Verfahren nach einem der vorstehenden Ansprüche, **dadurch gekennzeichnet, daß** man Einsatzstoffe und -geräte trocken einsetzt und Lösungsmittel, die eingesetzten Verbindungen der Formel 2 bis 4 und die Hilfsbase vorgetrocknet bzw. frisch destilliert verwendet.

8. Verfahren nach einem der den vorstehenden Ansprüche, **dadurch gekennzeichnet, daß** vor dem Start der Wassergehalt der Reaktionslösung unter 0,5 %, bevorzugt unter 0,1 %, besonders bevorzugt unter 0,001 % liegt.

9. Verfahren nach einem der Ansprüche 1 bis 6, **dadurch gekennzeichnet, daß** man vor der Umsetzung das organische Lösungsmittel, das schon eine Verbindung der Formel 3 oder 4 und ganz oder teilweise die Hilfsbase enthält und einen Wassergehalt unter 0,5 %, bevorzugt unter 0,2 %, besonders bevorzugt unter 0,05 % besitzt, mit Zusätzen, wie Natriumsulfat, Calciumchlorid (wasserfrei oder eine Hydratform), Phosphorpentoxid, Molekularsieb oder Aluminiumoxid in seinen verschiedenen Formen, behandelt.

## Claims

1. Process for the preparation of oxazaphosphorine-2-amines of the general formula 1 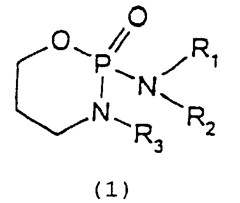 in which R₁ can be H, 2-bromoethyl, 2-chloroethyl, 2-hydroxyethyl or 2-mesyloxyethyl, R₂ can be H or 2-chloroethyl and R₃ can be H, 2-bromoethyl or 2-chloroethyl, or R₁ and R₂, together with the linked N atom, form an aziridine ring, R₁, R₂ and R₃ not simultaneously being H,
**characterized in that** a phosphoryl halide of the general formula 2,
an amine of the general formula 3
and an amine of the general formula 4 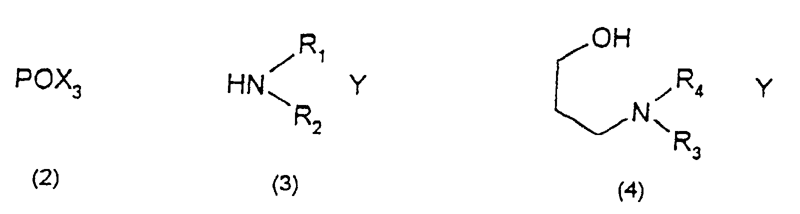 in which R₁, R₂ and R₃ have the same meaning as in formula 1, R₄ can be H, or R₃ and R₄, together with the linked N atom, form an aziridine ring, X is chlorine or bromine, X only being bromine if R₁ or R₃ is 2-bromoethyl, and Y has no meaning or can be hydrogen chloride or hydrogen bromide,
are reacted with an auxiliary base as an acid-binding agent, such as pyridine, triethylamine, alkali metal and alkaline earth metal carbonates, in the presence of inert solvents or diluents or mixtures, where, for example, halogenated and nonhalogenated hydrocarbons, aromatic and aliphatic hydrocarbons, such as, in particular, toluene, chloroform and dichloromethane, aliphatic and cyclic ethers, such as, in particular, diethyl ether and tert-butyl methyl ether, dioxane, tetrahydrofuran, are employed, with minimization of the effects of water and alcohols and without isolation of an intermediate compound.

2. Process according to Claim 1, **characterized in that**, in the first step, one compound each of the formulae 2 and 3 and, in the second step, a compound of the formula 4 is employed, R₁ not being 2-hydroxyethyl if the compound of the formula 3 is employed in the first step.

3. Process according to Claim 1 or 2, **characterized in that** a compound of the general formula 3 is initially introduced as a salt together with a compound of the formula 2 and the auxiliary base is slowly added with cooling.

4. Process according to Claim 2 or 3, **characterized in that** the compound of the formula 4 is employed instead of the compound of the formula 3 and the compound of the formula 3 is employed instead of the compound of the formula 4.

5. Process according to Claim 1, **characterized in that** the compounds of the formulae 3 and 4 are condensed simultaneously or in a mutually displaced manner with a compound of the formula 2.

6. Process according to any of the above claims, **characterized in that** the compound N-(2-chloroethyl)-3-hydroxypropylamine hydrochloride which contains N-(2-chloroethyl)-3-chloropropylamine hydrochloride is employed as a starting compound.

7. Process according to any of the above claims, **characterized in that** substances and equipment are employed in dry form and solvents, the compounds of the formulae 2 to 4 employed and the auxiliary base are used in predried or freshly distilled form.

8. Process according to any of the above claims, **characterized in that** before the start the water content of the reaction solution is below 0.5%, preferably below 0.1%, particularly preferably below 0.001%.

9. Process according to one of Claims 1 to 6, **characterized in that** before the reaction the organic solvent, which already contains a compound of the formula 3 or 4 and, completely or partially, the auxiliary base and has a water content of below 0.5%, preferably below 0.2%, particularly preferably below 0.05%, is treated with additives, such as sodium sulphate, calcium chloride (anhydrous or a hydrate form), phosphorus pentoxide, molecular sieve or alumina in its various forms.

## Revendications

1. Procédé de préparation d'oxazaphosphorin-2-amines de formule générale 1 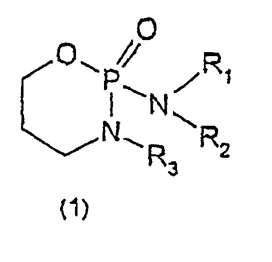 dans laquelle R₁ peut représenter H, un 2-bromoéthyle, un 2-chloroéthyle, un 2-hydroxyéthyle, un 2-mésyloxéthyle, R₂ peut représenter H et un 2-chloroéthyle et R₃ peut représenter H, un 2-bromoéthyle, un 2-chloroéthyle, ou R₁ et R₂ forment, conjointement avec l'atome d'azote lié, un noyau aziridine, R₁, R₂ et R₃ ne représentant pas simultanément H,
**caractérisé en ce qu'**un halogénure de phosphoryle de formule générale 2,
une amine de formule générale 3,
et une amine de formule générale 4, 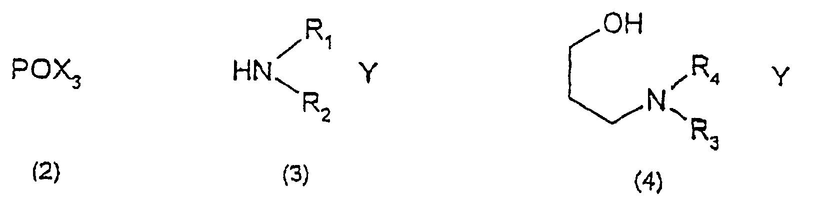 dans lesquelles R₁, R₂ et R₃ présentent la même signification que dans la formule 1, R₄ peut représenter H, ou R₃ et R₄ forment, conjointement avec l'atome d'azote lié, un noyau aziridine, X représente un chlore et un brome, X ne représentant un brome que si R₁ ou R₃ représente un 2-bromoéthyle, et Y ne présente aucune signification ou peut représenter un chlorure d'hydrogène et un bromure d'hydrogène,
sont mis à réagir avec une base auxiliaire en tant qu'agent de liaison aux acides, tel que la pyridine, la triéthylamine, des carbonates de métaux alcalins et de métaux alcalinoterreux, en présence de solvants ou de diluants inertes ou de leurs mélanges, en utilisant, par exemple, des hydrocarbures halogénés et non halogénés, des hydrocarbures aromatiques et aliphatiques, tels qu'en particulier le toluène, le chloroforme et le dichlorométhane, des éthers aliphatiques et cycliques, tels qu'en particulier l'éther diéthylique et l'éther tert-butylméthylique, le dioxane, le tétrahydrofurane, en minimisant l'influence de l'eau et des alcools et sans isoler un composé intermédiaire.

2. Procédé selon la revendication 1, **caractérisé en ce que** dans la première étape, un composé chacun de formules 2 et 3, et dans la deuxième étape, un composé de formule 4 sont utilisés, R¹ ne représentant pas un 2-hydroxyéthyle si le composé de formule 3 est utilisé dans la première étape.

3. Procédé selon la revendication 1 ou 2, **caractérisé en ce qu'**un composé de formule générale 3 est initialement introduit en tant que sel conjointement avec un composé de formule 2 et la base auxiliaire est ajoutée lentement en refroidissant.

4. Procédé selon la revendication 2 ou 3, **caractérisé en ce que** le composé de formule 4 est utilisé au lieu du composé de formule 3 et le composé de formule 3 est utilisé au lieu du composé de formule 4.

5. Procédé selon la revendication 1, **caractérisé en ce que** les composés de formules 3 et 4 sont condensés, simultanément ou de façon décalée l'un par rapport à l'autre, avec le composé de formule 2.

6. Procédé selon l'une quelconque des revendications précédentes, **caractérisé en ce que** l'on utilise en tant que composé de départ, le composé chlorhydrate de N-(2-chloroéthyl)-3-hydroxypropylamine qui contient du chlorhydrate de N-(2-chloroéthyl)-3-chloropropylamine.

7. Procédé selon l'une quelconque des revendications précédentes, **caractérisé en ce que** les substances et les appareils sont utilisés à sec et les solvants, les composés utilisés de formules 2 à 4 et la base auxiliaire sont employés sous forme préséchée ou fraîchement distillée.

8. Procédé selon l'une quelconque des revendications précédentes, **caractérisé en ce que**, avant de commencer, la teneur en eau de la solution réactionnelle est inférieure à 0,5 %, de préférence inférieure à 0,1 %, particulièrement préférablement inférieure à 0,001 %.

9. Procédé selon l'une quelconque des revendications 1 à 6, **caractérisé en ce que**, avant la réaction, le solvant organique qui contient déjà un composé de formule 3 ou 4 et la totalité ou une partie de la base auxiliaire, et présente une teneur en eau inférieure à 0,5 %, de préférence inférieure à 0,2 %, particulièrement préférablement inférieure à 0,05 %, est traité avec des additifs, tels que le sulfate de sodium, le chlorure de calcium (anhydre ou une forme hydratée), le pentoxyde de phosphore, un tamis moléculaire ou l'oxyde d'aluminium sous ses diverses formes.
